# FASCICULE DE BREVET EUROPEEN

(11) **EP 0 775 102 B1**
(45) Date de publication et mention de la délivrance du brevet: **05.04.2000**
(21) Numéro de dépôt: 96913678.7
(22) Date de dépôt: 28.05.1996
(51) Int. Cl.: C07C 49/647, C07C 45/74, C07C 45/67, C07C 69/738

(54) **PROCEDE POUR L'OBTENTION DE CETONES CYCLOALIPHATIQUES INSATUREES**
VERFAHREN ZUR HERSTELLUNG VON UNGESÄTTIGTEN CYCLOALIFATISCHEN KETONEN
PROCESS FOR OBTAINING UNSATURATED CYCLOALIPHATIC KETONES

(30) Priorité: 08.06.1995 CH 167695
(43) Date de publication de la demande: 28.05.1997
(73) Titulaire: FIRMENICH SA, 1211 Genève 8 (CH)
(72) Inventeur: NAEF, Ferdinand, CH-1227 Carouge (CH); DECORZANT, René, CH-1213 Onex (CH)
(74) Mandataire: Salvaterra-Garcia, Maria de Lurdes
(86) Numéro de dépôt international: IB9600499
(87) Numéro de publication internationale: WO9641789

(56) Documents cités:
- US-A- 2 069 861
- CANADIAN JOURNAL OF CHEMISTRY, vol. 61, 1983, OTTAWA CA, pages 288-297, XP000579894 E. PIERS ET AL.: "Five-membered ring spiro-annulation via thermal rearrangement of enol silyl ethers of 2-(cyclopropylmethylene)cycloalkanones. A formal total synthesis of some spirovetivane-type sesquiterpenoids"

## Description

### Domaine technique

La présente invention a trait au domaine de la synthèse organique. En particulier, elle concerne un procédé pour la préparation de cétones cycloaliphatiques insaturées de formule 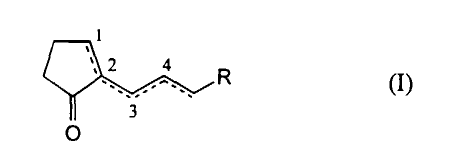 possédant deux doubles liaisons conjugées dans les positions 1 et 3, ou 2 et 4, telles qu'indiquées par les pointillés, et dans laquelle R sert à définir un radical alkyle de Cl à C3.

### Technique antérieure

Les composés de formule (I) sont des composés utiles à titre d'intermédiaires pour la préparation de dérivés jasmoniques dotés de propriétés odorantes fort appréciées parmi lesquels il convient de mentionner tout particulièrement le dihydrojasmonate de méthyle (Hédione®, marque enregistrée de Firmenich SA). Depuis sa découverte, ce dernier composé a fait l'objet de nombreuses synthèses [voir Edouard P. Demole, dans Fragrance Chemistry, Ed. E. Theimer, Academic Press, 1982] et l'intérêt que lui portent les parfumeurs s'est accru ces dernières années suite à la mise au point de méthodes de préparation permettant l'obtention de l'isomère cis préférentiel.

La méthode employée pour la préparation industrielle du dihydrojasmonate de méthyle est caractérisée par une première étape qui consiste en l'addition aldolique entre le pentanal et la cyclopentanone [voir brevet US 3,158,644 et Helv. Chim. Acta 20, 1474 (1937)], une réaction qui conduit à la formation de 2-pentylidényl-cyclopentanone, lequel composé est ensuite isomérisé en 2-pentyl-cyclopent-2-énone. Ce dernier composé, une fois additionné de malonate diméthylique et décarboxylé, conduit à un mélange de dihydrojasmonate de méthyle essentiellement constitué par l'isomère cyclanique trans.

Le cis-dihydrojasmonate de méthyle a été par contre préparé suivant la littérature par hydrogénation du 3-oxo-2-pentyl-cyclopent-2-ène-acétate de méthyle en présence de méthylate d'aluminium [DE-OS 2 162 820]. D'autres méthodes font état de distillation isomérisante du composé trans en présence d'un carbonate de métal alcalin ou alcalino-terreux. L'inconvénient majeur de tels procédés réside dans le fait qu'ils ne permettent d'obtenir le composé désiré que sous forme de mélange dont le contenu en l'isomère cis ne dépasse généralement pas 30%.

### Exposé de l'invention

La présente invention apporte une solution nouvelle au problème posé par une préparation industrielle tout à la fois simple et économique tant du dihydrojasmonate de méthyle, sous forme d'isomère cis ou trans, que de son homologue inférieur ou nor-méthyl dihydrojasmonate.

Le procédé de l'invention est dirigé vers la préparation d'une cyclopentanone diénique de formule (I), plus particulièrement sous leur forme isomérique de formule 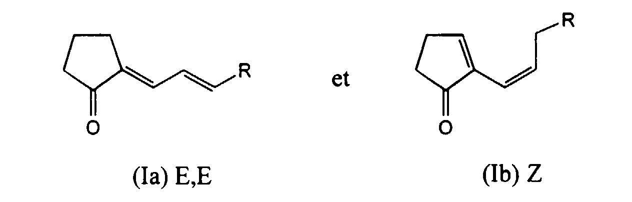 Nous avons constaté que l'addition aldolique en milieu basique d'un aldéhyde de formule R-CH=CH-CHO, dont la double liaison est de configuration (E), sur la cyclopentanone donnait lieu à la formation des diénones (Ia) et non pas, comme on aurait pu s'y attendre, aux carbinols allyliques de formule

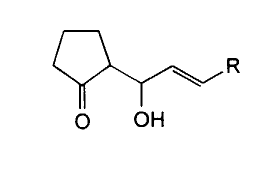

Lorsque les cétones diéniques (la), dont la configuration est de type (E,E), sont soumises à traitement thermique à une température comprise entre environ 200° et 450°C, elles se transforment en leurs isomères (Ib) possédant une double liaison endocyclique et une liaison double en position 1 de la chaîne latérale de configuration (Z). Or, c'est précisément dans cette configuration particulière que les composés obtenus par le procédé de l'invention réagissent de préférence avec le malonate de diméthyle pour l'étape suivante du procédé pour l'obtention du dihydrojasmonate de méthyle, ou de son homologue inférieur, suivant le schéma réactionnel que voici :

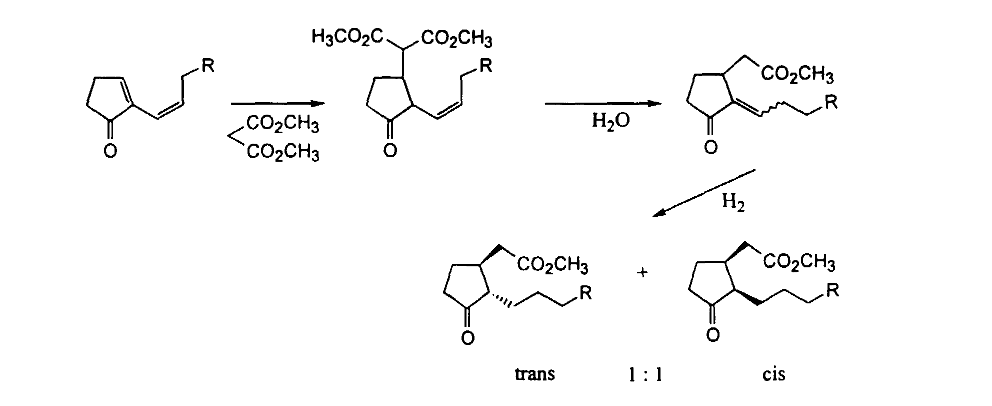

Tant l'addition du malonate de diméthyle que sa décarboxylation ont lieu selon des méthodes analogues à celles décrites dans la littérature [voir brevet US 3,158,644 et DE-OS 2 162 820].

Quant à l'hydrogénation, elle peut être conduite en présence de palladium sur charbon actif pour fournir un mélange équimoléculaire de dihydrojasmonate ou de nor-dihydrojasmonate de méthyle, sous leurs formes isomériques trans et cis.

Un des intérêts majeurs de l'approche synthétique suggérée par la présente invention réside dans le fait que, grâce aux cétones cycloaliphatiques préparées par le procédé décrit, l'on peut préparer, par le choix judicieux de la méthode d'hydrogénation, aussi bien des mélanges riches en l'isomère cis que ceux contenant essentiellement l'isomère trans.

La présente invention a donc pour objet un procédé pour l'obtention de cétones cycloaliphatiques de formule 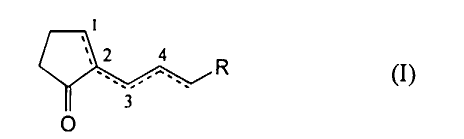 possédant deux doubles liaisons conjuguées dans les positions 1 et 3 ou 2 et 4 telles qu'indiquées par les pointillés, et dans laquelle R sert à définir un radical méthyle ou éthyle, le procédé étant caractérisé en ce qu'on additionne à la cyclopentanone, en présence d'un agent basique, un aldéhyde de formule 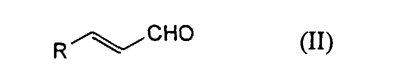 dont la double liaison est de configuration (E) et dans laquelle R a le sens indiqué ci-dessus, pour fournir une cyclopentanone diénique de formule 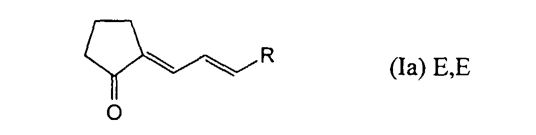 et l'on soumet, le cas échéant, la cyclopentanone ainsi formée à un traitement thermique pour donner une cyclopenténone de formule

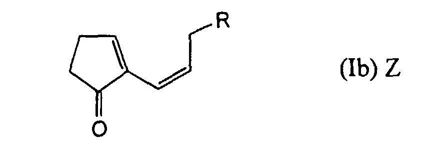

Parmi les composés (Ia) et (Ib) ainsi obtenus, certains constituent des entités chimiques nouvelles. Tel est le cas de la 2-(E,2'Z)-but-2'-énylidène-cyclopentanone, de la 2-(E,2'E)-pent-2'-énylidène-cyclopentanone, de la 2-(Z,2'E)-pent-2'-énylidène-cyclopentanone et de la 2-(1'Z)-pent-1'-ényl-cyclopent-2-énone, lesquels composés font également l'objet de l'invention.

La présente invention a également trait aux diesters carboxyliques de formule 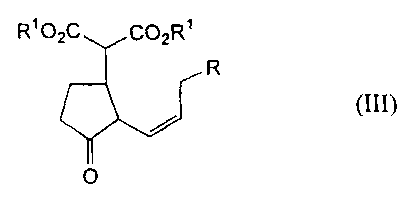 dans laquelle R a le sens indiqué plus haut à la formule (I) et R¹ représente un alkyle inférieur. Il s'agit de composés nouveaux obtenus à l'aide de méthodes similaires à celles connues par addition de malonate de dialkyle sur une cyclopentanone de formule (Ib) en présence d'un agent basique tel un alkoxyde d'un métal alcalin.

Tel qu'illustré à l'aide du schéma réactionnel donné plus haut, les diesters (III) sont des composés intermédiaires utiles pour la préparation de dérivés jasmoniques parfumants.

Selon le procédé de l'invention, l'addition de l'aldéhyde(II) sur la cyclopentanone a lieu en présence d'un agent basique. Comme agent basique, on utilise de préférence un hydroxyde ou un alkoxyde de métal alcalin, tel par exemple l'hydroxyde ou le méthylate de sodium. Il s'agit de bases courantes pour ce type de réaction.

L'étape suivante, qui consiste en l'isomérisation des cyclopentanones diéniques (Ia) obtenus, essentiellement sous forme d'isomères de configuration (E,E), en leurs isomères (Ib) de configuration (1'Z), s'effectue par traitement thermique. La température préférentielle pour un tel traitement se situe entre environ 200° et 450°C.

La réaction a lieu dans un four ordinaire ou tout simplement dans un tube, de préférence en quartz, chauffé à la température choisie et le cas échéant balayé par un courant de gaz inerte, l'azote ou l'argon par exemple. Le produit de départ est introduit graduellement à l'une des extrémités du tube, tandis qu'à l'autre extrémité le produit désiré est recueilli par condensation des vapeurs résultantes. Sa purification peut s'effectuer par l'une des méthodes usuelles, telle la chromatographie sur colonne ou la distillation fractionnée.

Les aldéhydes (E)-insaturés, employés comme produits de départ dans le procédé d'obtention de cétones cycloaliphatiques (I) selon l'invention, sont des produits commerciaux d'origines diverses [voir par exemple : Fluka AG, Buchs, Suisse].

L'invention est illustrée de manière plus détaillée par les exemples suivants dans lesquels les températures sont indiquées en degrés centigrades et les abréviations ont le sens usuel dans l'art.

### Manières de réaliser l'invention

### Exemple 1

### 2-(E,2'E)-Pent-2'-énylidène-cyclopentanone

a. 180 ml de soude caustique 1M ont été ajoutés goutte à goutte à 450 g (5,36 moles) de cyclopentanone maintenus sous agitation à environ 5-10°. Le mélange devient jaune au cours de l'addition. A cette même température, on a ensuite additionné en 1h30 au mélange réactionnel 150 g (1,78 moles) de (E)-pent-2-énal. La réaction est exothermique et la coloration passe du jaune au brun. L'agitation a été maintenue pendant 4h supplémentaires tandis que la température est portée à environ 20°. Le mélange a été extrait à l'éther (3x300 ml) et les extraits organiques ont été lavés avec une solution aqueuse d'HCl, neutralisés avec du NaHCO₃, de l'eau et enfin séchés. Par évaporation et distillation du résidu obtenu (525 g), on a obtenu 227 g d'une fraction à Eb. 87°/0,5-0,7 hPa.

Le contenu en la cétone diénique désirée est de 88% (rend. 74%).
- RMN (¹H, 360MHz, CDCl₃) :: 1,06(t, J=7,5Hz, 3H) ; 1,96(txt, J=8 et 7,5Hz, 2H) ; 2,23(qxd, J=7,5 et 6Hz, 2H) ; 2,34(t, J=8Hz, 2H) ; 2,69(t finement divisé, J=7,5Hz, 2H) ; 6,17(dxd, J=15 et 10,5Hz, 1H) ; 6,24(txd, J=15 et 6Hz, 1H) ; 6,92 (d finement divisé, J=10,5Hz, 1H) δ ppm
- RMN (¹³C, 90,5MHz, CDCl₃) :: 207,9(s) ; 147,4(d) ; 134,9(s) ; 131,9(d) ; 125,9(d) ; 38,6(t) ; 27,1(t) ; 26,5(t) ; 19,9(t) ; 13,0(q) δ ppm
- SM :: 150(M⁺, 13) : 135(2), 121(100), 107(2), 91(10), 79(25), 77(15), 65(5), 55(4), 39(8), 27(4).

### 2-(1'Z)-Pent-1-ényl-cyclopent-2-énone

b. 215 g (pureté 88%) de 2-(E,2'E)-pent-2'-énylidène-cyclopentanone ont été introduits dans une colonne en quartz de 100 x 0,8 cm chauffée à 350° et balayée par un flux d'azote de 6 l/h. L'introduction a lieu au moyen d'une seringue à une vitesse d'environ 12-15g/h. Le produit résultant est récupéré à 20°.

La colonne a été ensuite rincée avec 30 ml de cyclohexane et le condensat est concentré sous vide pour fournir un mélange 7:3 de 2-(l'Z)-pent-1'-ényl-cyclopent- 2-énone et de produit de départ. Par distillation sur résidu, on a obtenu 192 g d'un mélange contenant 60% de 2-(l'Z)-pent-1'-ényl-cyclopent-2-énone accompagné par 23% de produit de départ ainsi qu'une certaine quantité (12%) de deux de ses isomères de configuration, soit un rendement de 73%.

Le mélange obtenu peut être utilisé sans purification pour l'étape suivante. Le cas échéant, le 2-(1'Z)-pent-1'-ényl-cyclopent-2-énone peut être purifié par distillation fractionnée ou par chromatographie sur colonne.

Les données spectrales d'un échantillon purifié par chromatographie sur colonne de silice (Chromagel 35-70 µ ; 57 g) et un mélange de cyclohexane/éther 95:5 étaient les suivantes :
- RMN (¹H, 360MHz, CDCl₃) :: 0,94(t, J=7,5Hz, 3H) ; 1,47(qxt, J=7,5 et 7,5Hz, 2H) ; 2,19(txd finement divisé, J=7,5 et 7Hz, 2H) ; 2,40(m, 2H) ; 2,67(m, 2H) ; 5,80(txd, J=12 et 7Hz, 1H) ; 6,04(d finement divisé, J=12Hz, 1H) ; 7,53(t, J=3Hz, 1H) δ ppm
- RMN (¹³C, 90,5MHz, CDCl₃) :: 208,9(s) ; 158,0(d) ; 141,1(s) ; 136,9(d) ; 117,6(d) ; 33,9(t) ; 31,8(t) ; 26,9(t) ; 22,7(t) ; 13,9(q) δ ppm
- SM :: 150(M⁺, 77) : 135(34), 121(73), 117(17), 108(25), 91(54), 79(100), 77(62), 65(22), 55(24), 39(32), 27(19).

### Exemple 2

### [2-(Pent-(1'Z)-ényl)-3-oxo-1-cyclopentyl]-malonate de diméthyle

37,5 g (pureté 74% ; 0,25 moles) de 2-(1'Z)-pent-1'-ényl-cyclopent-2-énone ont été ajoutés goutte à goutte et sous agitation à 5-10° (2h) sur un mélange de 33 g (0,25 moles) de malonate de méthyle, 80 ml de méthanol et 1,7 g de méthylate de sodium à 30% dans le méthanol.

L'agitation est poursuivie pendant 4h à 5-10° avant que le mélange soit acidifié par l'addition de 10 ml d'acide acétique et concentré sous vide à 55°/200 hPa.

Le mélange concentré est dilué dans de l'eau et de l'éther. La phase organique est lavée à l'eau (2x), séchée sur du sulfate de magnésium et concentrée à l'aide d'un évaporateur rotatif pour fournir 73 g de produit brut.

Par distillation, on a obtenu 32,2 g du diester désiré ayant Eb. 120-145°/0,5 hPa (rend. 45%).

Un échantillon analytique a été obtenu par chromatographie sur colonne de silice (Chromagel 35-70µ ; 60 g) ; éluant : cyclohexane/éther 9:1 à 1:1.

Ses caractères analytiques étaient les suivants :
- RMN (¹H, 360MHz, CDCl₃) :: 0,92(t, J=7,5Hz, 3H) ; 1,40(qxt, J=7,5 et 7,5Hz, 2H) ; 1,76(m, 1H) ; 2,00(m, 2H) ; 2,28(m, 2H) ; 2,45(m, 1H) ; 2,63(m, 1H) ; 3,10(dxd, J=11 et 10Hz, 1H) ; 3,49(d, J=7Hz, 1H) ; 3,70(s, 3H) ; 3,75(s, 3H) ; 5,03(dxd finement divisé, J=11 et 10Hz, 1H) ; 5,75(txd, J=11 et 7Hz, 1H) δ ppm
- RMN (¹³C, 90,5MHz, CDCl₃) :: 215,7(s) ; 168,6(s) ; 168,3(s) ; 136,2(d) ; 124,1(d); 54,1(d) ; 52,6(d) ; 52,5(q) ; 52,4(q) ; 43,3(d) ; 37,4(t) ; 29,9(t) ; 24,9(t) ; 22,8(t) ; 13,9(q) δ ppm
- SM :: 282(M⁺, 6) : 264(1), 251(2), 233(1), 219(9), 205(1), 191(5), 177(3), 163(4), 151(95), 150(100), 133(93), 121(46), 117(13), 109(28), 101(19), 93(37), 79(63), 67(27), 59(37), 55(32), 41(30), 27(21).

### Exemple 3

### 2-(E,2'E)-But-2'-énylidène-cyclopentanone

a. 200 ml de soude caustique 1M ont été ajoutés goutte à goutte à 504 g (6 moles) de cyclopentanone maintenus sous agitation à environ 20°. Le mélange devient jaune au cours de l'addition.

140 g (2 moles) de crotonaldéhyde ont été ensuite ajoutés goutte à goutte à 5-10° en 1 1/2h et la coloration du mélange passe au brun clair, tandis que l'agitation a été poursuivie pendant 3h supplémentaires en maintenant la température du mélange à 20°. Après extraction à l'éther (3x300 ml), on a séparé la phase organique et les extraits éthérés combinés ont été lavés à l'HCl (1N), neutralisés avec du NaHCO₃, lavés avec une solution aqueuse saturée de NaCI et enfin séchés sur du MgSO₄.

Par évaporation dans un évaporateur rotatif sous vide, on a obtenu 562 g de concentrat qui ont été ensuite distillés pour fournir une fraction de 207 g à Eb. 56-73°/0,6 hPa. Une nouvelle distillation sur une colonne de type Widmer a fourni une fraction ayant Eb. 60-62°/0,6 hPa de 179 g de la cyclopentanone désirée ayant une pureté de 88% (rend. 58%).

Ses caractères analytiques étaient les suivants :
- RMN (¹H, 360MHz, CDCl₃) :: 1,89(d, J=5Hz, 3H) ; 1,96(txt, J=8 et 7Hz, 2H) ; 2,34(t, J=8Hz, 2H) ; 2,68(t finement divisé, J=7Hz, 2H) ; 6,20(m, 2H) ; 6,90(d finement divisé, J=8Hz, 1H) δ ppm
- RMN (¹³C, 90,5MHz, CDCl₃) :: 207,8(s) ; 140,6(d) ; 134,7(s) ; 131,6(d) ; 128,3(d) ; 38,6(t) ; 27,0(t) ; 19,9(t) ; 19,1(q) δ ppm
- SM :: 136(M⁺, 29) : 121(100), 107(3), 103(1), 91(12), 79(59), 74(1), 65(10), 55(4), 51(10), 39(19), 27(10)

Parmi les composés secondaires figurait la 2-(E,2'Z)-but-2'-énylidène-cyclopentanone dont les caractères spectraux étaient les suivants :
- RMN (¹H, 360MHz, CDCl₃) :: 1,90(d, J=8Hz, 3H) ; 1,96(m, 2H) ; 2,36(t, J=8Hz, 2H) ; 2,68 (t finement divisé, J=6Hz, 2H) ; 6,11 (m, 2H) ; 7,27(d finement divisé, J=12Hz, 1H) δ ppm
- RMN (¹³C, 90,5MHz, CDCl₃) :: 208,0(s) ; 137,1(d) ;136,5(s) ; 126,0(d) ; 125,8(d) ; 38,7(t) ; 26,9(t) ; 19,8(t) ; 14,1(q) δ ppm
- SM :: 136(M⁺, 29) : 121(100), 107(4), 103(1), 91(11), 79(45), 65(5), 51(3), 39(4), 27(1)
ainsi que la 2-(Z,2'E)-but-2'-énylidène-cyclopentanone :
- RMN (¹H, 360MHz, CDCl₃) :: 1,85(d finement divisé, J=8Hz, 3H) ; 1,90(txt, J=8 et (8Hz, 2H) ; 2,34(t, J=8Hz, 2H) ; 2,63(t, J=8Hz, 2H) ; 5,99(dxq, J=8 et J=16Hz, 1H) ; 6,33(d, J=12Hz, 1H) ; 7,53(dxd finement divisé, J=8, 12 et 16Hz, 1H) δ ppm
- RMN (¹³C, 90,5MHz, CDCl₃) :: 207,8(s) ; 139,0(d) ; 136,3(d) ; 132,4(s) ; 128,0(d) ; 40,6(t) ; 31,8(t) ; 20,5(t) ; 18,6(q) δ ppm
- SM :: 136(M⁺, 19) : 121(100), 107(3), 103(1), 91(10), 79(29), 65(4), 51(3), 39(3), 27(1)

### b. 2-(1'Z)-but-1'-ényl-cyclopent-2-énone

85 g (pureté 88%) de 2-(E,2'E)-but-2'-énylidène-cyclopentanone ont été introduits dans une colonne en quartz, de 100 cm de longueur et 0,8 cm de diamètre, chauffée à 350° et balayée par un flux d'azote de 6 l/h.

La vitesse d'introduction était de 12-15 g/h. Le produit a été recueilli à 20°. Le condensat se présente sous forme d'un mélange 7:3 de 2-(l'Z)-but-l'-ényl-cyclopent-2-énone et de produit de départ.

La colonne en quartz a été rincée avec 30 ml de cyclohexane et le condensat a été concentré sous vide. Par distillation répétée sur colonne Widmer, on a obtenu 58 g de la cétone désirée ayant une pureté de 68%, Eb. 84-86°/4 hPa.

Le produit a été purifié par distillation.

Les caractères analytiques du produit obtenu étaient les suivants :
- RMN (¹H, 360MHz, CDCl₃) :: 1,05(t, J=7,5Hz, 3H) ; 2,23(qxd finement divisé, J=7,5 et 7Hz, 2H) ; 2,41(m, 2H) ; 2,68(m, 2H) ; 5,78(txd, J=12 et 5Hz, 1H) ; 6,00(d finement divisé, J=12Hz, 1H) ; 7,52(t, J=3Hz, 1H) δ ppm
- RMN (¹³C, 90,5MHz, CDCl₃) :: 209,0(s) ; 158,2(d) ; 141,0(s) ; 138,6(d) ; 117,0(d) ; 33,9(t) ; 26,9(t) ; 23,0(t) ; 13,9(q) δ ppm
- SM :: 136(M⁺, 99), 121(74), 117(15), 107(23), 103(10), 91(54), 79(100), 77(70), 74(3), 65(27), 55(18), 51(23), 39(44), 27(19).

### Exemple 4

### [2-But-(1'Z)-ényl)-3-oxo-1-cyclopentyl]-malonate de diméthyle

32 g (0,24 moles) de 2-(1'Z)-but-1'-ényl-cyclopent-2-énone (pureté 71%) ont été ajoutés goutte à goutte sous agitation à 5-10° en 75 min à un mélange de 31 g (0,24 moles) de malonate de diméthyle dans 80 ml de méthanol et 1,7 g de méthylate de sodium à 30% dans le méthanol.

L'agitation a été poursuivie pendant 4 h à 5-10°.

Le mélange réactionnel a été acidifié par addition de 10 ml d'acide acétique avant d'être concentré au moyen d'un évaporateur rotatif à 55°/200 hPa.

Le concentrat obtenu a été dilué dans de l'eau et de l'éther, puis la phase organique a été lavée à l'eau (2x), séchée sur du MgSO₄ et concentrée pour fournir 68 g de résidu.

Une distillation de ce produit a fourni l'ester malonique désiré à Eb. 130°/0,5 hPa.

Un échantillon analytique a été purifié par redistillation.

Ses caractères analytiques étaient les suivants :
- RMN (¹H, 360MHz, CDCl₃) :: 0,99(t, J=7,5Hz, 3H) ; 1,75(m, 1H) ; 2,05(m, 2H) ; 2,27(m, 2H) ; 2,43(m, 1H) ; 2,63(m, 1H) ; 3,09(dxd, J=11 et 10Hz, 1H) ; 3,49(d, J=7Hz, 1H) ; 3,70(s, 3H) ; 3,75(s, 3H) ; 4,99(dxd finement divisé, J=11 et 10Hz, 1H); 5,73(txd, J=11 et 7Hz, 1H) δ ppm
- RMN (¹³C, 90,5MHz, CDCl₃) :: 215,7(s) ; 168,6(s) ; 168,3(s) ; 137,8(d) ; 123,5(d) ; 54,2(d) ; 52,6(d) ; 52,5(q) ; 52,4(q) ; 43,2(d) ; 37,4(t) ; 25,0(t) ; 21,1(t) ; 14,2(q) δ ppm
- SM :: 268(M⁺, 3) : 237(1), 219(2), 205(10), 193(2), 187(2), 177(5), 163(3), 149(5), 137(100), 136(78), 133(46), 121(17), 107(8), 101(9), 93(20), 79(20), 69(10), 59(16), 55(8), 41(5), 29(4).

### Exemple 5

En procédant comme indiqué aux exemples précédents mais en faisant réagir l'hex-2-énal sur la cyclopentanone, on a obtenu la 2-(E,2'E)-hex-2'-énylidène-cyclopentanone dont les caractères analytiques étaient les suivants :
- RMN (¹H, 360MHz, CDCl₃) :: 0,93(t, J=7,5Hz, 3H) ; 1,46(qxt, J=7,5 et 7,5Hz, 2H) ; 1,96(txt, J=8 et 8Hz, 2H) ; 2,18(txd, J=7,5 et 7Hz) ; 2,34(t, J=8Hz) ; 2,68(t finement divisé, J=8Hz, 2H) ; 6,18(m, 2H) ; 6,90(d finement divisé, J=8Hz, 1H) δ ppm
- RMN (¹³C, 90,5MHz, CDCl₃) :: 207,9(s) ; 145,9(d) ; 134,8(s) ; 131,9(d) ; 127,0(d) ; 38,6(t) ; 35,5(t) ; 27,0(t) ; 22,1(t) ; 19,9(t) ; 13,7(q) δ ppm
- SM :: 164(M⁺, 10) : 149(1), 135(3), 121(100), 107(3), 91(13), 79(19), 77(21), 65(7), 55(5), 51(5), 39(12), 27(11)

Le composé ainsi obtenu a été traité comme indiqué à l'Exemple 3.b pour fournir la 2-(1'Z)-hex-1'-éthyl-cyclopent-2-énone :
- RMN (¹H, 360MHz, CDCl₃) :: 0,91(t, J=7,5Hz, 3H) ; 1,35(m, 2H) ; 1,42(m, 2H) ; 2,23(txd finement divisé, J=7,5 et 7Hz, 2H) ; 2,43(m, 2H) ; 2,68(m, 2H) ; 5,81(txd, J=12 et 7Hz, 1H) ; 6,03(d finement divisé, J=12Hz, 1H) ; 7,53(t, J=3Hz, 1H) δ ppm
- RMN (¹³C, 90,5MHz, CDCl₃) :: 209,0(s) ; 158,0(d) ; 141,1(s) ; 137,2(d) ; 117,4(d) ; 33,9(t) ; 31,6(t) ; 29,4(t) ; 26,9(t) ; 22,4(t) ; 13,9(q) δ ppm
- SM :: 164(M⁺, 60): 149(9), 135(42), 121(68), 117(15), 108(32), 96(34), 91(59), 79(100), 77(65), 65(24), 55(19), 39(40), 27(28)

Enfin, la transformation de ce composé en [2-hex-(1'Z)-ényl)-3-oxo-1-cyclopentyl]malonate de diméthyle est obtenue conformément à l'Exemple 4.

Son spectre RMN était le suivant :
- RMN (¹H, 360MHz, CDCl₃) :: 0,90(t, J=7,5Hz, 3H) ; 1,34(m, 4H) ; 1,75(m, 1H) ; 2,03(m, 2H) ; 2,27(m, 2H) ; 2,44(m, 1H) ; 2,64(m, 1H) ; 3,10(dxd, J=11 et 10Hz, 1H) δ ppm

## Revendications

1. Procédé pour l'obtention de cétones cycloaliphatiques de formule 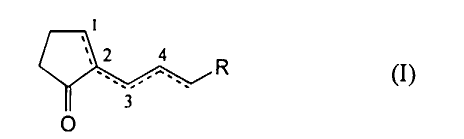 possédant deux doubles liaisons conjugées dans les positions 1 et 3, ou 2 et 4, telles qu'indiquées par les pointillés, et dans laquelle R sert à définir un radical alkyle de Cl à C3, caractérisé en ce qu'on additionne à la cyclopentanone, en présence d'un agent basique, un aldéhyde de formule 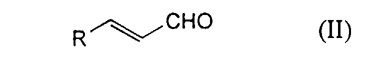 dont la double liaison est de configuration (E) et dans laquelle R a le sens indiqué ci-dessus, pour fournir une cyclopentanone diénique de formule 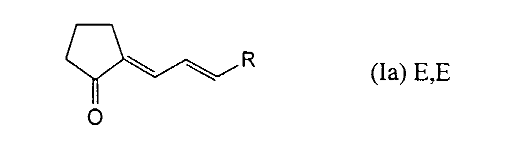 et l'on soumet, le cas échéant, la cyclopentanone ainsi formée à un traitement thermique pour donner une cyclopenténone de formule

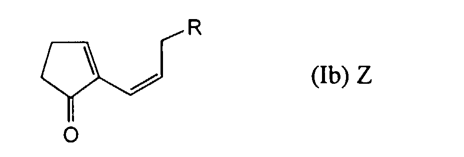

2. Procédé selon la revendication 1 caractérisé en ce que le traitement thermique de la cyclopentanone diénique de formule (la) E,E s'effectue à une température comprise entre environ 200° et 450°C.

3. Composés de formule (la) et (Ib) tels que définis à la revendication 1, appartenant au groupe que voici :
2-(E,2'E)-hex-2'-énylidène-cyclopentanone,
2-(E,2'E)-pent-2'-énylidène-cyclopentanone et
2-(1'Z)-pent-1'-ényl-cyclopent-2-énone.

4. Utilisation d'une cyclopenténone de formule (Ib) Z telle que définie à la revendication 1, à titre de produit de départ pour la préparation de diesters carboxyliques de formule 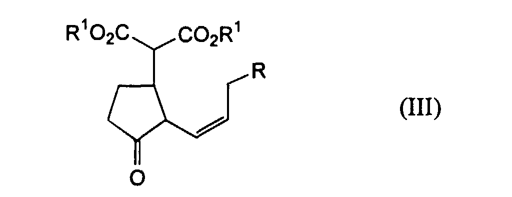 dans laquelle R représente un radical alkyle de C1 à C3 et R¹ définit un alkyle inférieur, caractérisé en ce que ladite cyclopenténone (Ib) Z est soumise à une réaction d'addition avec un malonate dialkylique.

5. Composés de formule (III) telle que définie à la revendication 4.

6. A titre de composé selon la revendication 5,
le [2-(pent-(1'Z)-ényl)-3-oxo-1-cyclopentyl]-malonate de diméthyle,
le [2-(but-(1'Z)-ényl)-3-oxo-1-cyclopentyl]-malonate de diméthyle, et
le [2-(hex-(1'Z)-ényl)-3-oxo-1-cyclopentyl]-malonate de diméthyle.

## Claims

1. Process for the preparation of cycloaliphatic ketones of formula 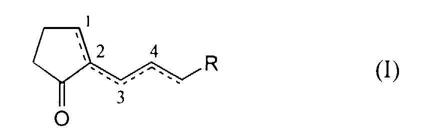 having two conjugated double bonds in positions 1 and 3, or 2 and 4, such as indicated by the dotted lines, and wherein R defines a C₁ to C₃ alkyl radical, said process being characterized in that there is added to the cyclopentanone, in the presence of a basic agent, an aldehyde of formula 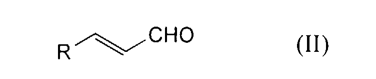 wherein the double bond is of (E) configuration and wherein R has the meaning indicated above, to provide a diene cyclopentanone of formula 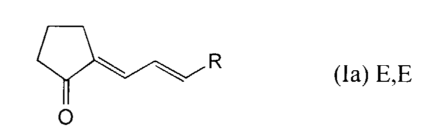 and, optionally, in that the cyclopentanone thus formed is subjected to a thermal treatment to provide a cyclopentenone of formula

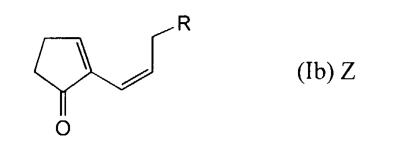

2. Process according to claim 1, characterized in that the thermal treatment of the diene cyclopentanone of formula (Ia) E,E takes place at a temperature comprised between about 200° and 450°C.

3. Compounds of formula (Ia) and (Ib) such as defined in claim 1, selected from the following group :
2-(E,2'E)-hex-2'-enylidene-cyclopentanone,
2-(E,2'E)-pent-2'-enylidene-cyclopentanone, and
2-('Z)-pent-1'-enyl-cyclopent-2-enone.

4. Use of a cyclopentenone of formula (Ib) Z such as defined in claim 1, as starting product for the preparation of carboxylic diesters of formula 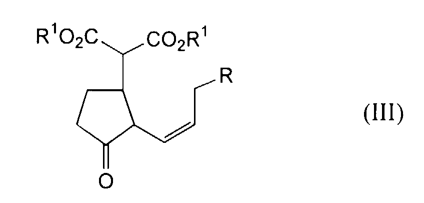 wherein R represents a C₁ to C₃ alkyl radical and R¹ defines a lower alkyl, characterized in that said cyclopentenone (Ib) Z is subjected to an addition reaction with a dialkyl malonate.

5. Compounds of formula (III) such as defined in claim 4.

6. As a compound according to claim 5,
dimethyl [2-(pent-(1'Z)-enyl)-3-oxo-1-cyclopentyl]-malonate,
dimethyl [2-(but-(1'Z)-enyl)-3-oxo-1-cyclopentyl]-malonate, and
dimethyl [2-(hex-(1'Z)-enyl)-3-oxo-1-cyclopentyl]-malonate.

## Patentansprüche

1. Verfahren zur Herstellung von cycloaliphatischen Ketonen mit der Formel 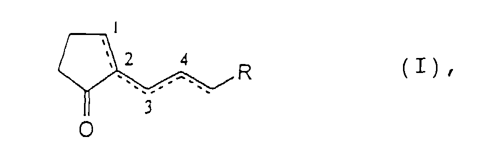 die in den Positionen 1 und 3 bzw. 2 und 4 zwei konjugierte Doppelbindungen aufweisen, wie gestrichelt angedeutet ist, und in der R dazu dient, einen C1-C3-Alkylrest zu definieren, dadurch gekennzeichnet, daß an Cyclopentanon in Gegenwart eines basischen Mittels ein Aldehyd mit der Formel 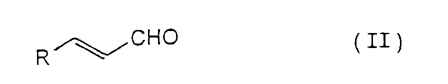 addiert wird, dessen Doppelbindung die (E)-Konfiguration aufweist, und in der R die oben angegebene Bedeutung besitzt, um ein Diencyclopentanon mit der Formel 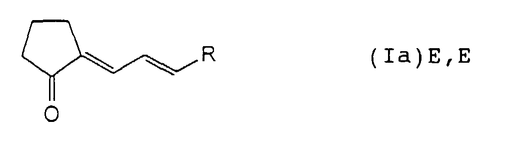 zur Verfügung zu stellen, und das auf diese Weise gebildete Cyclopentanon gegebenenfalls einer Wärmebehandlung unterzogen wird, wodurch ein Cyclopentenon mit der Formel 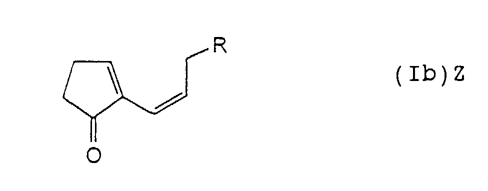 hergestellt wird.

2. Verfahren gemäß Anspruch 1, dadurch gekennzeichnet, daß die Wärmebehandlung des Diencyclopentanons mit der Formel (Ia)E,E bei einer zwischen ca. 200° und 450°C liegenden Temperatur durchgeführt wird.

3. Verbindungen mit der Formel (Ia) und (Ib) gemäß der Definition von Anspruch 1, die der folgenden Gruppe angehören:
2-(E,2'E)-Hex-2'-enyliden-cyclopentanon,
2-(E,2'E)-Pent-2'-enyliden-cyclopentanon und
2-(1'Z)-pent-1'-enyl-cyclopent-2-enon.

4. Verwendung eines Cyclopentenons mit der Formel (Ib)Z gemäß der Definition von Anspruch 1 als Ausgangsprodukt für die Herstellung von Carbonsäurediestern mit der Formel 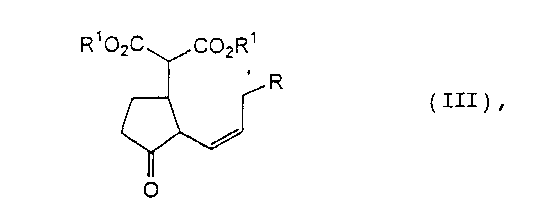 in der R für einen C1-C3-Alkylrest steht und R¹ ein niederes Alkyl definiert, dadurch gekennzeichnet, daß das Cyclopentenon (Ib)Z einer Additionsreaktion mit einem Malonsäuredialkylester unterzogen wird.

5. Verbindungen mit der Formel (III) gemäß der Definition von Anspruch 4.

6. Als Verbindung gemäß Anspruch 5,
[2-(Pent-(1'Z)-enyl)-3-oxo-1-cyclopentyl]-malonsäuredimethylester, [2-(But-(1'Z)-enyl)-3-oxo-1-cyclopentyl]-malonsäuredimethylester, und [2-(Hex-(1'Z)-enyl)-3-oxo-1-cyclopentyl]-malonsäuredimethylester.
